# EUROPÄISCHE PATENTSCHRIFT

(11) **EP 2 666 438 B1**
(45) Veröffentlichungstag und Bekanntmachung des Hinweises auf die Patenterteilung: **10.04.2019**
(21) Anmeldenummer: 13004153.6
(22) Anmeldetag: 18.04.2012
(51) Int. Cl.: A61C 13/12, A61C 13/00

(54) **Prothesenzahnträger**
Prosthetic tooth support
Support pour une prothèse dentaire

(30) Priorität: 16.05.2011 DE 102011101678
(43) Veröffentlichungstag der Anmeldung: 27.11.2013
(62) Teilanmeldung aus: 12721695.0
(73) Patentinhaber: Amann Girrbach AG, 6842 Koblach (AT)
(72) Erfinder: Noack, Falko, 6890 Lustenau (AT)
(74) Vertreter: Fechner, Thomas

(56) Entgegenhaltungen:
- EP-A1- 1 304 089
- DE-C- 403 973
- US-A1- 2002 125 619
- US-A1- 2007 190 492

## Beschreibung

Die vorliegende Erfindung betrifft einen Prothesenzahnträger mit zumindest einem Prothesenzahn, insbesondere mit mehreren Prothesenzähnen. Weiters betrifft die Erfindung auch Verfahren zur Herstellung eines Prothesenzahnträgers.

Dentale Voll- oder Teilprothesen bestehen grundsätzlich aus zumindest zwei unterschiedlichen Komponenten. Dies ist zum Einen eine Prothesenbasis, welche die fehlenden Zahnfleischanteile ersetzt. Zum Anderen sind dies die Prothesenzähne bzw. Ersatzzähne, welche als Ersatz für die fehlenden natürlichen Zähne des Patienten fungieren. Die Prothesenzähne sind mit der Prothesenbasis verbunden und haben sowohl therapeutische als auch ästhetische Funktionen. Prothesenzähne werden von verschiedenen Herstellern angeboten. Alle am Markt erhältlichen Prothesenzähne sind konfektionierte Formen, die in ihren Farben variieren. Es existieren am Markt in verschiedenen Größen vorkonfektionierte Zahnformen, welche vom Zahntechniker entsprechend bestimmter individueller Charakteristika des Patienten wie z.B. Platzangebot, Körpergröße, Geschlecht und Figur ausgewählt werden. Die am Markt erhältlichen Prothesenzähne werden für die Anwendung in Voll- und Teilprothesen im Patientengebiss weitestgehend unbearbeitet verwendet. Hierzu bleibt speziell der Kronenbereich der Prothesenzähne weitgehend unversehrt. Der Hals- bzw. Wurzelbereich der Prothesenzähne muss jedoch in den meisten Fällen auf Grund der unterschiedlichen Bisshöhen, also dem unterschiedlichen Abstand zwischen Ober- und Unterkiefer der einzelnen Patienten angepasst werden. Hierzu werden die Zahnhals- und Wurzelbereiche der Prothesenzähne eingekürzt. Dies wird beim Stand der Technik vom Zahntechniker frei Hand realisiert, wodurch einerseits Ungenauigkeiten, andererseits aber auch ein erheblicher Zeitaufwand entstehen, da die Prothesenzahnlänge immer wieder überprüft werden muss. Beim Einkürzen der Zähne versucht man möglichst viele Anteile des Zahnhalses unbearbeitet zu lassen und nur so wenig wie möglich einzukürzen bzw. abzutragen. Der Zahnhals- und Wurzelbereich des Prothesenzahnes dient als Verbindungsfläche zur Prothesenbasis, hat aber auch wichtige Funktionen für die Ästhetik des Prothesenzahns und damit der gesamten Prothese.

In der gesamten Zahntechnik hat in den letzten Jahren immer mehr die automatisierte bzw. rechnergesteuerte Herstellung von Zahnersatz Einzug gehalten. Insbesondere die Herstellung von Vollprothesen stellt aktuell ein Betätigungsfeld im Bereich der dentalen CAD-CAM Entwicklung dar. Dies betrifft sowohl das Design der Prothesenbasis als auch die Positionierung der einzelnen Prothesenzähne relativ zur Prothesenbasis. Aus diesem Grund werden für die am Markt erhältlichen Prothesenzähne auch bereits die Informationen über deren Form digitalisiert, also als Datensätze angeboten. Um die digital bzw. virtuell zusammengesetzte bzw. designte Vollprothese nun aber auch reell umsetzen zu können, müssen nach wie vor die am Markt erhältlichen Prothesenzähne eingekürzt werden, um in der Prothesenbasis entsprechend Platz zu finden.

Beim Stand der Technik werden die Prothesenzähne auf Prothesenzahnträgern am Markt angeboten, wobei die Prothesenzähne allerdings nur über eine Haftmasse leicht haftend auf den Prothesenzahnträger aufgeklebt sind. Die hierfür verwendeten Haftmittel dienen lediglich dazu, sicher zu stellen, dass die Prothesenzähne beim Transport nicht verloren gehen und zur besseren Ansicht positioniert sind. Die Haftmittel sind so schwach, dass die Prothesenzähne ohne Weiteres händisch vom Prothesenzahnträger entfernt werden können. Solche Zahnhalteplatten sind z. B. in der EP 1 243 233 A1 und der EP 1 304 089 A1 gezeigt.

In der US 2002/0125619 A1 wird ein Grünling ausgefräst und dann gesintert.

Aus der US2007/0190492A1 ist ein Prothesenzahnträger mit den Merkmalen des Oberbegriffs des Anspruchs 1 bekannt.

Es ergibt sich die Aufgabe, Prothesenzähne für einen automatisierten Bearbeitungsschritt sicher in an sich bekannten, mittels Datenverarbeitungseinrichtungen gesteuerten Fräseinrichtungen in der Weise positionieren zu können, dass die Zahnhals- und Wurzelbereiche der Prothesenzähne automatisiert in der gewünschten Art und Weise eingekürzt werden können.

Zur Lösung dieser Aufgabe wird ein Prothesenzahnträger gemäß Patentanspruch 1 vorgeschlagen.

Es ist somit eine Grundidee der Erfindung, den Prothesenzahn oder die Prothesenzähne jeweils mit ihrem Kronenbereich so in einer Trägerschicht des erfindungsgemäßen Prothesenzahnträgers einzubetten bzw. in dieser einzugießen, dass der Zahnhals- und Wurzelbereich der Prothesenzähne aus der Trägerschicht herausschaut und mittels geeigneter, beim Stand der Technik z.B. in Form von CNC-Fräsmaschinen bekannter Fräseinrichtungen bearbeitet bzw. gekürzt werden kann. Dies ermöglicht es, die Prothesenzähne in definierten Positionen in der Trägerschicht einzubetten. Der Fräsmaschinenadapter erlaubt es, den Prothesenzahnträger sicher und in einer definierten Position in oder an der Fräseinrichtung befestigen zu können. Der Fräsmaschinenadapter kann in einer Variante zapfenförmig von der Trägerschicht abstehend ausgebildet sein.

Im Sinne einer Vermeidung von Bedienungsfehlern weist der Fräsmaschinenadapter günstigerweise formschlüssige Indexierungen z.B. in seiner Oberfläche auf, damit er nur in einer einzigen bzw. eindeutigen Position in der Fräsmaschine befestigt werden kann. Solche Indexierungen sind an sich bekannt und können in Form von Kerben, Anschlägen, Polygonzügen und dergleichen ausgebildet sein.

Die definierten Positionen im Prothesenzahnträger und die digital bereits vorhandene oder gegebenenfalls durch Einscannen oder andere geeignete Mittel zu erstellende Information über die Zahnform können dann z. B. in einem, vorzugsweise auf einem einheitlichen Koordinatensystem basierenden, Datensatz abgespeichert und einer, die Fräseinrichtung steuernden Datenverarbeitungseinrichtung zugeführt werden.

Es besteht auch die Möglichkeit ein Set aus einem erfindungsgemäßen Prothesenzahnträger und einem Datensatz, wobei der Datensatz die Informationen über die Form und die Position des Prothesenzahns bzw. der Prothesenzähne in und am Prothesenzahnträger beinhaltet, vorzusehen.

Um einen sicheren Halt während der Bearbeitung zu gewährleisten, bietet es sich an, die Prothesenzähne in der Trägerschicht in einem entsprechend festen aber fräsbaren Material, beispielsweise Wachs oder Kunststoff, welches keine chemische Verbindung mit dem Prothesenzahn eingeht, einzubetten. Da die Trägerschicht die Funktion des In-Positionhaltens der Prothesenzähne vollständig ausfüllt, kann in einer Variante der Erfindung vorgesehen sein, dass ein Zahnhals- und Wurzelbereich des Prothesenzahnes bzw. der Prothesenzähne zumindest bereichsweise, vorzugsweise vollständig, frei einsehbar über die Trägerschicht übersteht. Der Kronenbereich des Prothesenzahns bzw. der Prothesenzähne ist zur Einbettung zumindest bereichsweise in die Trägerschicht eingegossen oder umspritzt. Er kann auch vollständig in die Trägerschicht eingegossen oder umspritzt sein.

Zur Herstellung eines solchen Prothesenzahnträgers sieht ein bevorzugtes Verfahren vor, dass der Prothesenzahn bzw. die Prothesenzähne mit dem Zahnhals- und Wurzelbereich in einer Hilfshaltevorrichtung positioniert wird bzw. werden und dann der Kronenbereich des Prothesenzahns bzw. der Prothesenzähne zumindest bereichsweise, gegebenenfalls vollständig, in der Trägerschicht eingegossen oder umspritzt wird bzw. werden.

Um die Prothesenzähne nach dem Einkürzen des Zahnhals- und Wurzelbereichs aus der Trägerschicht entnehmen zu können, weist diese günstigerweise eine Sollbruchstelle auf. Diese Sollbruchstelle kann bereits herstellerseitig oder vom Anwender mittels Fräseinrichtung in die Trägerschicht eingebracht werden.

Es muss aber nicht zwingend vorgesehen sein, dass die Zahnhals- und Wurzelbereiche des Prothesenzahnes bzw. der Prothesenzähne frei einsehbar sind. Alternativ kann auch vorgesehen sein, dass ein Zahnhals- und Wurzelbereich des Prothesenzahnes bzw. der Prothesenzähne zumindest bereichsweise, vorzugsweise vollständig, in eine Abdeckschicht des Prothesenzahnträgers eingegossen oder umspritzt, ist. Bei dieser Variante können der oder die Prothesenzähne also vollständig im Prothesenzahnträger verborgen sein. Dies stört die Bearbeitung bzw. das Einkürzen nicht, da die Positionen und die Formen der Prothesenzähne ja über den bereits genannten Datensatz bekannt sein können.

Ein bevorzugtes erfindungsgemäßes Verfahren zur Herstellung eines Prothesenzahnträgers, bei dem die Zahnhals- und Wurzelbereiche in der Abdeckschicht eingebettet sind, sieht vor, dass der Prothesenzahn bzw. die Prothesenzähne mit dem Kronenbereich in einer Hilfshaltevorrichtung positioniert wird bzw. werden und dann der Zahnhals- und Wurzelbereich des Prothesenzahnes bzw. der Prothesenzähne zumindest bereichsweise, vorzugsweise vollständig, in der Abdeckschicht des Prothesenzahnträgers eingegossen oder umspritzt wird bzw. werden und daran anschließend der Kronenbereich des Prothesenzahns bzw. der Prothesenzähne zumindest bereichsweise, gegebenenfalls vollständig, in die Trägerschicht eingegossen oder umspritzt wird bzw. werden.

Die Abdeckschicht sollte, sofern sie vorhanden ist, aus einem fräsbaren Material bestehen. Damit die fertig eingekürzten Prothesenzähne dann aus der Trägerschicht auch entfernt werden können, besteht diese günstigerweise ebenfalls aus einem fräsbaren Material. Grundsätzlich kommen dabei verschiedene Kunststoffe oder Wachse als solche Materialien in Frage. Im Sinne der Fräsbarkeit ist jedenfalls günstigerweise vorgesehen, dass die Trägerschicht und/oder die Abdeckschicht ein Material aufweist bzw. aufweisen oder daraus besteht bzw. daraus bestehen, welches eine Vickershärte zwischen 10 HV und 200 HV aufweist. Günstige Ausgestaltungsformen der Erfindung sehen jedenfalls vor, dass das Material der Trägerschicht zumindest so hart wie oder härter als das Material der Abdeckschicht ist.

Mit einem oben bereits genannten Set aus einem erfindungsgemäßen Prothesenzahnträger und dem zugeordneten Datensatz sieht ein Verfahren zur Bearbeitung zumindest eines Prothesenzahnes, vorzugsweise mehrerer Prothesenzähne, eines entsprechenden Sets vor, dass der Prothesenzahnträger des Sets mittels seines Fräsmaschinenadapters in eine Fräseinrichtung eingespannt wird und der Datensatz des Sets in eine Datenverarbeitungseinrichtung eingelesen wird und die Fräseinrichtung von der Datenverarbeitungseinrichtung zum Bearbeiten des Prothesenzahns oder der Prothesenzähne unter Berücksichtigung des Datensatzes angesteuert wird. Hierdurch wird es möglich, computergesteuert die einzukürzenden Bereiche des Zahnhals- und Wurzelbereichs der Prothesenzähne im Prothesenzahnträger gezielt anzufahren und auszufräsen. Dies ermöglicht es, am Computer durchgeführte digitale bzw. virtuelle Modelle einer dentalen Teil- oder Vollprothese in die reale Welt umzusetzen, indem hierfür die Prothesenzähne in der benötigten Art und Weise eingekürzt werden.

Zudem ist es möglich, auch zusätzliche Nuten oder Rillen in dem Prothesenzahn während des beschriebenen Frässchnittes einzuarbeiten, welche danach als Rotationsschutz oder zusätzliche Retention für den Prothesenzahn in der Prothesenbasis fungieren.

Sollte während der Konstruktion der Prothese ein Einkürzen der Prothesenzähne auch im Kronenbereich als notwendig ermittelt worden sein, so ist auch diese Manipulation der Zähne während des Fräsvorganges möglich, zumal die Trägerschicht, wie oben bereits ausgeführt, günstigerweise aus einem fräsbaren Material besteht.

Im Sinne einer besonders hohen Bedienerfreundlichkeit kann bei einem solchen Verfahren vorgesehen sein, dass der Datensatz im oder am Prothesenzahnträger, vorzugsweise im oder am Fräsmaschinenadapter, gespeichert wird und vor dem oder beim oder nach dem Einspannen des Prothesenzahnträgers in die Fräseinrichtung, vorzugsweise automatisch, von der Datenverarbeitungseinrichtung eingelesen wird. Der Prothesenzahnträger kann also selbst einen Datenträger wie z.B. einen Transponder, Balkencode oder dergleichen aufweisen, auf welchem der Datensatz abgespeichert ist. Dieser Datenträger kann am Fräsmaschinenadapter oder an anderer geeigneter Stelle am Prothesenzahnträger angeordnet sein. Die Datenverarbeitungseinrichtung kann dann den Datensatz automatisch oder nach Abfrage einlesen, sobald der Prothesenzahnträger in die Fräseinrichtung eingespannt ist.

Der Vollständigkeit halber sei darauf hingewiesen, dass der Datensatz des genannten erfindungsgemäßen Sets natürlich nicht zwingend am Prothesenzahnträger direkt angeordnet sein muss. Der Datensatz kann auch anderweitig gespeichert und in die Datenverarbeitungseinrichtung eingelesen werden. Hierzu stehen beim Stand der Technik zahlreiche, an sich bekannte Varianten zur Verfügung, welche hier nicht alle im Einzelnen aufgelistet werden müssen.

Der Vollständigkeit halber sei darauf hingewiesen, dass unter einem Prothesenzahn ein künstlicher Zahn zu verstehen ist, welcher in eine dentale Voll- oder Teilprothese eingebaut werden soll. Der Kronenbereich umfasst den äußeren Teilbereich des Prothesenzahnes, welcher bei einem gesunden natürlichen Zahn aus dem Zahnfleisch herausschaut. Der Kronenbereich umfasst die Kau- bzw. Okklusalfläche des Zahnes. Der Zahnhals- und Wurzelbereich eines natürlichen Zahnes ist bei gesundem Zahnfleisch in diesem verborgen. Im Falle eines Prothesenzahnes ist der Zahnhals- und Wurzelbereich bei fertiggestellter Teil- oder Vollprothese zumindest großteils in der Prothesenbasis verborgen. Der Zahnhals- und Wurzelbereich umfasst die Basalfläche des Prothesenzahns und dient der Befestigung des Prothesenzahns in einer entsprechenden Ausnehmung in der Prothesenbasis.

Weiters sei noch darauf hingewiesen, dass geeignete Fräseinrichtungen und Datenverarbeitungseinrichtungen beim Stand der Technik in Form von verschiedensten CAD- und CNC-Fräseinrichtungen mit entsprechender Rechneransteuerung bekannt sind und hier nicht weiter erläutert werden müssen.

Weitere Merkmale und Einzelheiten bevorzugter Ausgestaltungsformen der Erfindung werden anhand der nachfolgenden Figurenbeschreibung erläutert. Es zeigen:
- Fig. 1 und 2: beispielhaft beim Stand der Technik an sich bekannte Prothesenzähne;
- Fig. 3: die beim Stand der Technik an sich bekannte Anordnung dieser Prothesenzähne in einer im Querschnitt dargestellten Vollprothese;
- Fig. 4 und 5: Darstellungen zur Notwendigkeit des Einkürzens der Prothesenzähne;
- Fig. 6 bis 8: Beispiele eines erfindungsgemäßen Prothesenzahnträgers;
- Fig. 9: eine schematisierte Darstellung zur erfindungsgemäßen Bearbeitung der in einem erfindungsgemäßen Prothesenzahnträger angeordneten Prothesenzähne;
- Fig. 10 bis: 15schematisierte Darstellungen zur Herstellung eines erfindungsgemäßen Prothesenzahnträgers;
- Fig. 16 und 17: schematisierte Darstellungen zum Einkürzen der im erfindungsgemäßen Prothesenzahnträger angeordneten Prothesenzähne.

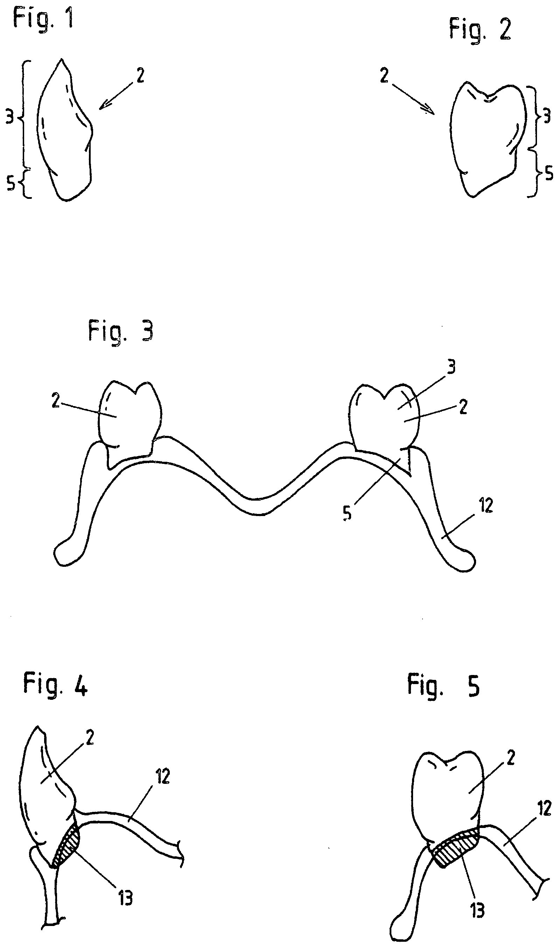

In Fig. 1 ist schematisiert ein beim Stand der Technik an sich bekannter und am Markt so auch erhältlicher Prothesenzahn 2 in Form eines Frontzahnes gezeigt. Fig. 2 zeigt beispielhaft einen Prothesenzahn 2 in Form eines Backenzahns. In beiden Figuren sind der Kronenbereich 3 und der Zahnhals- und Wurzelbereich 5 des jeweiligen Prothesenzahns 2 eingezeichnet. Fig. 3 zeigt einen Querschnitt durch eine Vollprothese. In der Prothesenbasis 12 sind in den entsprechenden Ausnehmungen die Prothesenzähne 2 mittels ihrer Zahnhals- und Wurzelbereiche 5 befestigt. Die Länge des Zahnhals- und Wurzelbereichs 5 gibt vor, wie weit der gesamte Prothesenzahn aus der Prothesenbasis 12 heraussteht. Das untere Ende des Zahnhals- und Wurzelbereichs 5 bildet mit der entsprechenden Gegenfläche der Prothesenbasis 12 einen entsprechenden Anschlag und die Abstützfläche für den Prothesenzahn 2. Bei den Prothesenzähnen 2 handelt es sich um vorgefertigte Produkte, welche am Markt in einer vorkonfektionierten Form und Größe angeboten werden. Die Prothesenbasis hingegen ist individuell an den Kiefer des jeweiligen Patienten anzupassen. Um auch die Länge des jeweiligen Prothesenzahnes 2 an die individuellen Bedürfnisse des Patienten anpassen zu können, ist es, wie eingangs bereits erläutert, notwendig, den jeweiligen Prothesenzahn an den in der Prothesenbasis zur Verfügung stehenden Platz in seiner Länge anzupassen. Hierzu muss ein Prothesenzahn 2 gegebenenfalls in seiner Länge gekürzt werden. Dies hat durch entsprechende Entfernung eines Teilbereichs 13 des Zahnhals- und Wurzelbereichs 5 zu erfolgen, wie dies schematisiert in den Fig. 4 und 5 dargestellt ist.

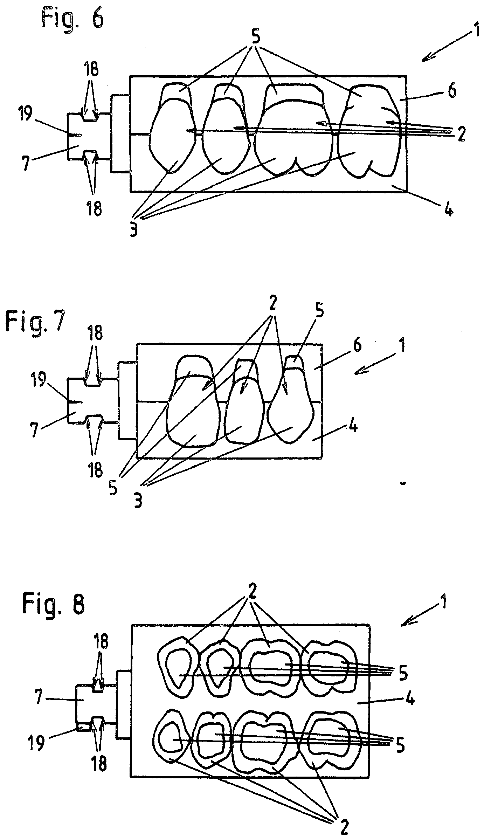

Erfindungsgemäß ist zur Automatisierung dieses Einkürzvorgangs die Verwendung von Prothesenzahnträgern 1 vorgesehen, welche beispielhaft in den Fig. 6 bis 8 dargestellt sind. Die Prothesenzahnträger 1 können einen aber auch mehrere Prothesenzähne 2 beinhalten. Bevorzugt umfasst ein Prothesenzahnträger vier oder acht Backenzähne oder drei oder sechs Frontzähne. In Fig. 6 ist eine Seitenansicht auf einen Prothesenzahnträger 1 mit insgesamt 8 als Backenzahnersatz vorgesehenen Prothesenzähnen 2 dargestellt. In der Seitenansicht gemäß Fig. 6 sind nur vier dieser Prothesenzähne 2 zu sehen. Fig. 8 zeigt eine Draufsicht auf die Zahnhals- und Wurzelbereiche 5 dieser Prothesenzähne 2. In Fig. 8 sind daher auch alle acht Prothesenzähne 2 zu sehen.

Fig. 7 zeigt eine zu Fig. 6 entsprechende Seitenansicht, in der drei als Frontzahnersatz vorgesehene Prothesenzähne 2 angeordnet sind. Die Prothesenzähne 2 sind jeweils mit ihren Kronenbereichen 3 in die Trägerschicht 4 des jeweiligen Prothesenzahnträgers 1 eingebettet und dort festgehalten. Die Abdeckschicht 6, welche die Zahnhals- und Wurzelbereiche 5 einbettet ist optional. In den Fig. 6 und 7 ist sie vorhanden, sie kann aber auch weggelassen sein, sodass die Zahnhals- und Wurzelbereiche 5 dann frei von außen einsehbar über die Trägerschicht 4 überstehen. Der Vollständigkeit halber wird darauf hingewiesen, dass die Trägerschicht 4 und die Abdeckschicht 6 in den Fig. transparent dargestellt sind, damit man die Prothesenzähne 2 sieht. In Realität muss diese Transparenz natürlich nicht gegeben sein. Die Prothesenzähne können in der Trägerschicht 4 und der gegebenenfalls vorhandenen Abdeckschicht 6 auch nicht von außen sichtbar verborgen sein.

An den in den Fig. 6 bis 8 dargestellten Prothesenzahnträgern 1 ist jeweils ein Fräsmaschinenadapter 7 befestigt, der dem Einspannen des Prothesenzahnträgers 1 in einer an sich bekannten Fräseinrichtung 10 dient. Die Fräsmaschinenadapter 7 sind günstigerweise so in ihrer Form ausgebildet bzw. indiziert, dass sie ausschließlich in einer einzigen Position in einer entsprechenden Aufnahme der Fräsmaschine befestigt werden können. In den Fig. 6 bis 8 ist dies durch die für eine formschlüssige Befestigung in der Fräseinrichtung 10 vorgesehene Schulterflächen 18 und durch die Indexnase 19 erreicht.

Im Sinne des Sets sind die Positionen und die Formen der Prothesenzähne 2 im Prothesenzahnträger 1 bekannt und in Form eines Datensatzes 9 abgespeichert. Hierdurch wird es der mittels der Datenverarbeitungseinrichtung 11 gesteuerten Fräseinrichtung 10 möglich, die Zahnhals- und Wurzelbereiche 5 der jeweiligen Prothesenzähne 2 in Abhängigkeit der jeweiligen Anforderungen, an die individuell auszugestaltende Voll- oder Teilprothese angepasst, zu bearbeiten bzw. einzukürzen.

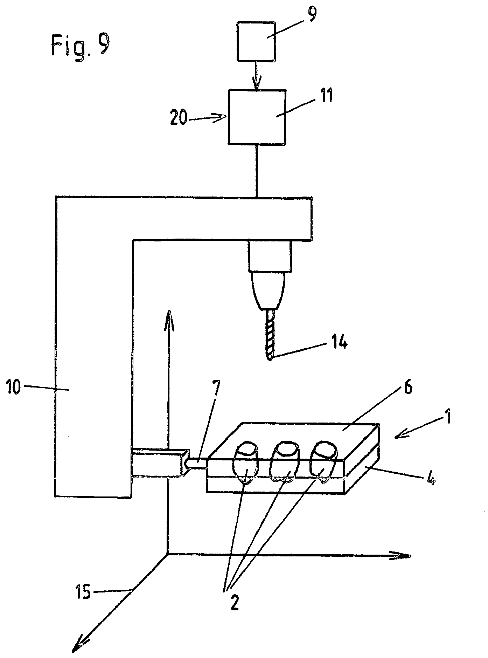

Fig. 9 zeigt schematisiert eine mittels der Datenverarbeitungseinrichtung 11 kontrollierte und angesteuerte Fräseinrichtung 10. Geeignete Fräseinrichtungen 10 sind z.B. als CNC-Fräsmaschinen beim Stand der Technik bekannt. Der schematisiert in Fig. 9 dargestellte Prothesenzahnträger 1 ist mittels seines Fräsmaschinenadapters 7 in der Fräseinrichtung 10 eingespannt. Für das Bearbeiten bzw. Abfräsen der Zahnhals- und Wurzelbereiche 5 sowie der gegebenenfalls vorhandenen Abdeckschicht 6 des Prothesenzahnträgers 1 ist ein schematisiert dargestellter Fräskopf 14 vorgesehen. Fräskopf 14 und Prothesenzahnträger 1 können in an sich bekannter Weise so relativ zueinander positioniert und verstellt werden, dass der Fräskopf 14 alle gewünschten Bereiche des Prothesenzahnträgers 1 und insbesondere der Zahnhals- und Wurzelbereiche 5 der im Prothesenzahnträger 1 angeordneten Prothesenzähne 2 in der gewünschten Art bearbeiten bzw. abfräsen kann. Gesteuert wird der Fräsvorgang der Fräseinrichtung mittels der Datenverarbeitungseinrichtung 11. Die Position und Form der Prothesenzähne 2 im jeweiligen Prothesenzahnträger 11 enthält der Datensatz 9, welcher über eine geeignete Art und Weise von der Datenverarbeitungseinrichtung 11 eingelesen wird. Über an sich bekannte Möglichkeiten werden der Datenverarbeitungseinrichtung 11 zusätzlich noch die Sollwerte 20 zugeführt, welche vorgeben, um welches Maß der oder die Prothesenzähne 2 bearbeitet bzw. eingekürzt werden muss bzw. müssen. Mit einer in Fig. 9 schematisiert dargestellten Anordnung kann das eingangs erwähnte Verfahren zur Bearbeitung des oder der Prothesenzähne durchgeführt werden. Fig. 9 stellt schematisiert ein gemeinsames Koordinatensystem 15 dar, auf dessen Basis die Datenverarbeitungseinrichtung 11 arbeitet und auf dessen Basis auch die Informationen zur Position und Form des Prothesenzahns 2 bzw. der Prothesenzähne 2 im Prothesenzahnträger 1 im Datensatz 9 abgespeichert sind. Mit dem Fräskopf 14 können nicht nur die Zahnhals- und Wurzelbereiche 5 und die gegebenenfalls vorhandene Abdeckschicht 6 bearbeitet bzw. abgefräst werden. Mittels des Fräskopfes 14 kann bevorzugt auch eine entsprechende Sollbruchstelle in der Trägerschicht 4 eingefräst werden, um nach Fertigstellung der Bearbeitung der Zahnhals- und Wurzelbereiche 5 und gegebenenfalls der Zahnkronenbereiche die Prothesenzähne 2 aus dem Prothesenzahnträger 1 entnehmen zu können. Solche Sollbruchstellen können aber natürlich auch bereits vorab vom Hersteller im Prothesenzahnträger 1 eingearbeitet werden.

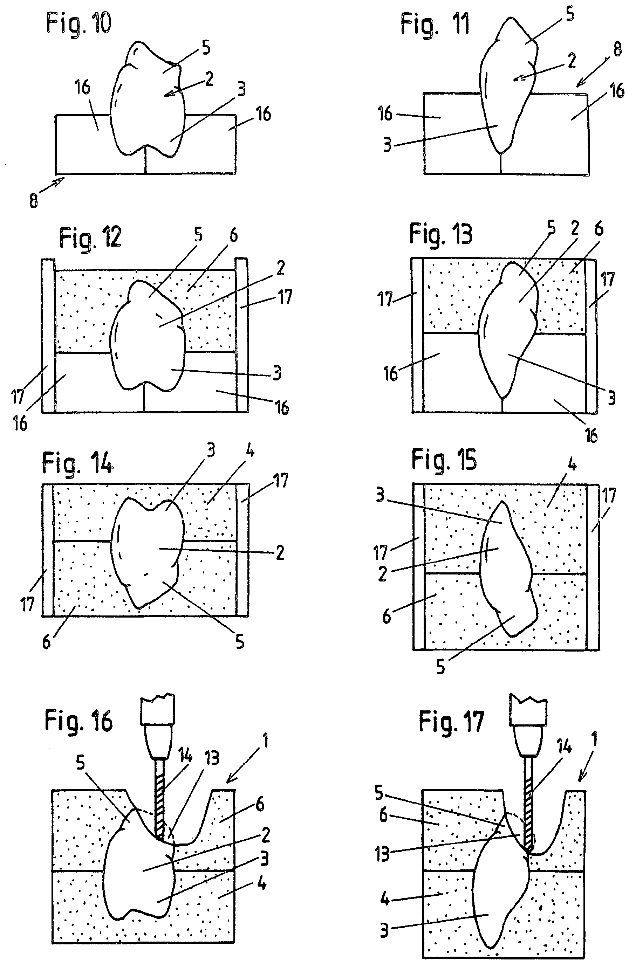

Die Fig. 10 bis 15 veranschaulichen beispielhaft ein Verfahren zur Herstellung eines erfindungsgemäßen Prothesenzahnträgers 1. Die Fig. 10, 12 und 14 zeigen einzelne Schritte am Beispiel eines als Backenzahn ausgebildeten Prothesenzahns 2, die Fig. 11, 13 und 15 zeigen dasselbe beispielhaft anhand eines als Frontzahn vorgesehenen Prothesenzahns 2. Die Prothesenzähne 2 sind, wie eingangs bereits erläutert, in der dargestellten Form am Markt erhältlich. Sie werden mittels einer geeigneten Hilfshaltevorrichtung in eine definierte Position gebracht und dort gehalten. Dies ist in Fig. 10 und 11 dargestellt. In beiden Figuren bestehen die Hilfshaltevorrichtungen 8 aus jeweils zwei Haltebacken 16, welche auseinander und aufeinander zu gefahren werden können. In den Fig. 10 und 11 ist der jeweilige Prothesenzahn 2 bereits entsprechend zwischen den Haltebacken 16 in der jeweiligen Hilfshaltevorrichtung 8 positioniert und gehalten, wodurch die Position des jeweiligen Prothesenzahns 2 im zugrundeliegenden Koordinatensystem 15 bekannt ist. Die Hilfshaltevorrichtungen 8 können unterschiedlich viele Aufnahmen für unterschiedlich viele Prothesenzähne 2 aufweisen. Die Anzahl ist günstigerweise jeweils auf den herzustellenden Prothesenzahnträger und die in ihm vorgesehene Anzahl von Prothesenzähnen 2 abzustimmen.

Der Vollständigkeit halber sei darauf hingewiesen, dass bei einer entsprechenden Ausformung der Prothesenzähne 2 die Hilfshaltevorrichtungen 8 nicht unbedingt zwei- bzw. mehrteilig ausgeführt sein müssen. Im Allgemeinen ist dies aber günstig. Beispielsweise können die Zähne in einteiligen Formen auch mittels Ansaugmechanismen in der Form fixiert werden.

In den Fig. 12 und 13 erfolgt nun der nächste Verfahrensschritt. Zunächst wird der Prothesenzahn 2 samt Hilfshaltevorrichtung 8 mittels des Mantels 17 ummantelt. Anschließend wird die Abdeckschicht 6 gegossen oder umspritzt, womit ein über die Haltevorrichtung 8 hervorstehender Teilbereich der Prothesenzähne 2 in die Abdeckschicht 6 eingebettet wird. In der Abdeckschicht 6 sollte sich vorzugsweise der gesamte Zahnhals- und Wurzelbereich 5 des Prothesenzahns bzw. der Prothesenzähne 2 befinden. In der Abdeckschicht 6 können sich aber auch Teile des Kronenbereichs 3 befinden. Der Kronenbereich 3 muss letztendlich nur so weit in der Trägerschicht 4 eingebettet sein, dass der jeweilige Prothesenzahn 2 bei der Bearbeitung mittels Fräskopf 14 ausreichend festgehalten ist. Nach Aushärten der Abdeckschicht 6, welche vorzugweise aus einem geeigneten fräsbaren Kunststoff oder Wachs besteht, kann die Hilfshaltevorrichtung 8 entfernt werden. Die Prothesenzähne 2 sind dann in der Abdeckschicht 6 positionstreu gehalten. Nach Entfernen der Hilfshaltevorrichtung 8 kann dann die Trägerschicht 4 gegossen bzw. durch Umspritzen erzeugt werden, sodass der Kronenbereich des Prothesenzahns 2 bzw. der Prothesenzähne 2 in der Trägerschicht 4 eingebettet ist. Nach gegebenenfalls notwendigem Aushärten des Materials der Trägerschicht 4 kann der Mantel 17 entfernt werden. Anschließend kann, sofern benötigt, der Fräsmaschinenadapter 7 noch am Prothesenzahnträger 1 angebracht werden. In den Fig. 16 und 17 ist dann noch schematisiert dargestellt, wie der so fertiggestellte Prothesenzahnträger 1 nach dem Einspannen in der Fräseinrichtung 10 mittels des Fräskopfes 14 bearbeitet wird, indem im benötigten Umfang die Abdeckschicht 6 und der zu entfernende Teilbereich 13 des Zahnhals- und Wurzelbereichs 5 nach den Vorgaben der Datenverarbeitungseinrichtung 11 abgefräst werden.

Die Fig. 10 bis 17 beziehen sich auf ein Ausführungsbeispiel bei dem der Prothesenzahnträger 1 sowohl eine Trägerschicht 4 als auch eine Abdeckschicht 6 aufweisen.

Wie bereits eingangs erläutert, ist die Abdeckschicht 6 aber nicht unbedingt zwingend notwendig. Um einen Prothesenzahnträger 1 ohne Abdeckschicht 6 herzustellen, kann das anhand der Fig. 10 bis 15 erläuterte Herstellungsverfahren dahingehend abgewandelt werden, dass die vorkonfektionierten Prothesenzähne 2 nicht mit ihrem Kronenbereich 3 sondern mit ihrem Zahnhals- und Wurzelbereich 5 in einer geeignet ausgeformten, die Positionsvorgabe beinhaltenden Hilfshaltevorrichtungen 8 positioniert werden. Anschließend kann ein entsprechender Mantel 17 an der Hilfshaltevorrichtung 8 angebracht werden. Danach wird dann direkt die Trägerschicht 4 gegossen oder durch Umspritzen aufgebracht und damit die frei über die Hilfshaltevorrichtung 8 überstehenden Bereiche des Kronenbereichs 3 der Prothesenzähne 2 eingebettet. Nach Entfernen des Mantels 17 und der Hilfshaltevorrichtung 8 ist dann eine Trägerschicht 4 mit darin angeordneten Prothesenzähnen 2 hergestellt, bei der die Zahnhals- und Wurzelbereiche 5 über die Trägerschicht 4 frei überstehen und entsprechend mittels eines geeigneten Fräskopfes 14 in einer Fräseinrichtung 10 bearbeitet werden können.

### zu den Hinweisziffern:

- 1: Prothesenzahnträger
- 2: Prothesenzahn
- 3: Kronenbereich
- 4: Trägerschicht
- 5: Zahnhals- und Wurzelbereich
- 6: Abdeckschicht
- 7: Fräsmaschinenadapter
- 8: Hilfshaltevorrichtung
- 9: Datensatz
- 10: Fräseinrichtung
- 11: Datenverarbeitungseinrichtung
- 12: Prothesenbasis
- 13: zu entfernender Teilbereich
- 14: Fräskopf
- 15: Koordinatensystem
- 16: Haltebacken
- 17: Mantel
- 18: Schulterfläche
- 19: Indexnase
- 20: Sollwerte

## Patentansprüche

1. Prothesenzahnträger (1) mit zumindest einem Prothesenzahn (2), insbesondere mit mehreren Prothesenzähnen (2), **dadurch gekennzeichnet, dass** ein Kronenbereich (3) des Prothesenzahns (2) bzw. der Prothesenzähne (2) zumindest bereichsweise in eine Trägerschicht (4) eingegossen oder umspritzt ist und an der Trägerschicht (4) ein Fräsmaschinenadapter (7) des Prothesenzahnträgers (1) zur Befestigung des Prothesenzahnträgers (1) während eines Fräsvorgangs in einer Fräseinrichtung (10) befestigt ist.

2. Prothesenzahnträger (1) nach Anspruch 1, **dadurch gekennzeichnet, dass** ein Zahnhals- und Wurzelbereich (5) des Prothesenzahnes (2) bzw. der Prothesenzähne (2) zumindest bereichsweise, vorzugsweise vollständig, frei einsehbar über die Trägerschicht (4) übersteht.

3. Prothesenzahnträger (1) nach Anspruch 1, **dadurch gekennzeichnet, dass** ein Zahnhals- und Wurzelbereich (5) des Prothesenzahnes (2) bzw. der Prothesenzähne (2) zumindest bereichsweise, vorzugsweise vollständig, in eine Abdeckschicht (6) des Prothesenzahnträgers (1) eingebettet, vorzugsweise eingegossen, ist.

4. Verfahren zur Herstellung eines Prothesenzahnträgers (1) nach einem der Ansprüche 2 oder 3, **dadurch gekennzeichnet, dass** der Prothesenzahn (2) bzw. die Prothesenzähne (2) mit dem Zahnhals- und Wurzelbereich (5) in einer Hilfshaltevorrichtung (8) positioniert wird bzw. werden und dann der Kronenbereich (3) des Prothesenzahns (2) bzw. der Prothesenzähne (2) zumindest bereichsweise in der Trägerschicht (4) eingegossen oder umspritzt wird bzw. werden.

5. Verfahren zur Herstellung eines Prothesenzahnträgers (1) nach Anspruch 3, **dadurch gekennzeichnet, dass** der Prothesenzahn (2) bzw. die Prothesenzähne (2) mit dem Kronenbereich (3) in einer Hilfshaltevorrichtung (8) positioniert wird bzw. werden und dann der Zahnhals- und Wurzelbereich (5) des Prothesenzahnes (2) bzw. der Prothesenzähne (2) zumindest bereichsweise, vorzugsweise vollständig, in der Abdeckschicht (6) des Prothesenzahnträgers (1) eingebettet, vorzugsweise eingegossen, wird bzw. werden und daran anschließend der Kronenbereich (3) des Prothesenzahns (2) bzw. der Prothesenzähne (2) zumindest bereichsweise in die Trägerschicht (4) eingegossen oder umspritzt wird bzw. werden.

## Claims

1. A prosthetic tooth support (1) having at least one prosthetic tooth (2), in particular having a plurality of prosthetic teeth (2), **characterised in that** at least regions of a crown region (3) of the prosthetic tooth (2) or the prosthetic teeth (2) are cast into a support layer (4) or the latter is injection-moulded therearound and a milling machine adapter (7) of the prosthetic tooth support (1) is secured to the support layer (4) in order to secure the prosthetic tooth support (1) during a milling procedure in a milling device (10).

2. A prosthetic tooth support (1) according to claim 1, **characterised in that** at least regions, preferably the entirety, of a tooth neck and root region (5) of the prosthetic tooth (2) or the prosthetic teeth (2) protrude beyond the support layer (4) in a freely visible manner.

3. A prosthetic tooth support (1) according to claim 1, **characterised in that** at least regions, preferably the entirety, of a tooth neck and root region (5) of the prosthetic tooth (2) or the prosthetic teeth (2) are embedded, preferably cast, into a cover layer (6) of the prosthetic tooth support (1).

4. A method of producing a prosthetic tooth support (1) according to one of claims 2 and 3, **characterised in that** the prosthetic tooth (2) or the prosthetic teeth (2) is/are positioned in an auxiliary holding device (8) by means of the tooth neck and root region (5) and then at least regions of the crown region (3) of the prosthetic tooth (2) or the prosthetic teeth (2) are cast in the support layer (4) or the latter is injection-moulded therearound.

5. A method of producing a prosthetic tooth support (1) according to claim 3, **characterised in that** the prosthetic tooth (2) or the prosthetic teeth (2) is/are positioned in an auxiliary holding device (8) by means of the crown region (3) and then at least regions, preferably the entirety, of the tooth neck and root region (5) of the prosthetic tooth (2) or the prosthetic teeth (2) are embedded, preferably cast, in the cover layer (6) of the prosthetic tooth support (1) and subsequently at least regions of the crown region (3) of the prosthetic tooth (2) or the prosthetic teeth (2) are cast into the support layer (4) or the latter is injection-moulded therearound.

## Revendications

1. Support de dents prothétiques (1) comportant au moins une dent prothétique (2), en particulier plusieurs dents prothétiques (2), **caractérisé en ce qu'**une zone de couronne (3) de la dent prothétique (2), ou des dents prothétiques (2), est scellée ou encapsulée au moins par zones dans une couche support (4) et un adaptateur de fraiseuse (7) du support de dents prothétiques (1) est fixé sur la couche support (4) pour fixer le support de dents prothétiques (1) dans un dispositif de fraisage (10) pendant une opération de fraisage.

2. Support de dents prothétiques (1) selon la revendication 1, **caractérisé en ce qu'**une zone de collet et de racine (5) de la dent prothétique (2), ou des dents prothétiques (2), dépasse bien visible au-dessus de la couche support (4) au moins par zones, de préférence entièrement.

3. Support de dents prothétiques (1) selon la revendication 1, **caractérisé en ce qu'**une zone de collet et de racine (5) de la dent prothétique (2), ou des dents prothétiques (2), est noyée, de préférence scellée, au moins par zones, de préférence entièrement, dans une couche de recouvrement (6) du support de dents prothétiques (1).

4. Procédé de fabrication d'un support de dents prothétiques (1) selon l'une des revendications 2 ou 3, **caractérisé en ce que** la dent prothétique (2), ou les dents prothétiques (2), est, ou sont, positionnée(s) avec la zone de collet et de racine (5) dans un dispositif de maintien auxiliaire (8), puis la zone de couronne (3) de la dent prothétique (2), ou des dents prothétiques (2), est, ou sont, scellée(s) ou encapsulée(s), au moins par zones, dans la couche support (4).

5. Procédé de fabrication d'un support de dents prothétiques (1) selon la revendication 3, **caractérisé en ce que** la dent prothétique (2), ou les dents prothétiques (2), est, ou sont, positionnée(s) avec la région de couronne (3) dans un dispositif de maintien auxiliaire (8), puis la zone de collet et de racine (5) de la dent prothétique (2), ou des dents prothétiques (2), est, ou sont, noyée(s), de préférence scellée(s), au moins par zones, de préférence entièrement, dans la couche de recouvrement (6) du support de dents prothétiques (1), puis à la suite la zone de couronne (3) de la dent prothétique (2), ou des dents prothétiques (2), est, ou sont, scellée(s) ou encapsulée(s), au moins par zones, dans la couche support (4).
